(19) Europäisches Patentamt
European Patent Office
Office européen des brevets

(11) **EP 2 814 854 B1**

(12) **EUROPÄISCHE PATENTSCHRIFT**

(45) Veröffentlichungstag und Bekanntmachung des Hinweises auf die Patenterteilung:
**23.01.2019 Patentblatt 2019/04**

(21) Anmeldenummer: **13702480.8**

(22) Anmeldetag: **04.02.2013**

(51) Int Cl.:
**C08F 220/06** (2006.01)   **A61L 15/00** (2006.01)

(86) Internationale Anmeldenummer:
**PCT/EP2013/052153**

(87) Internationale Veröffentlichungsnummer:
**WO 2013/120722 (22.08.2013 Gazette 2013/34)**

(54) **WASSERABSORBIERENDE POLYMERPARTIKEL MIT HOHER QUELLGESCHWINDIGKEIT UND HOHER PERMEABILITÄT**

WASSERABSORBIERENDE POLYMERPARTIKEL MIT HOHER QUELLGESCHWINDIGKEIT UND HOHER PERMEABILITÄT

PARTICULES POLYMÈRES HYDROPHILES PRÉSENTANT UNE VITESSE DE GONFLEMENT ET UNE PERMÉABILITÉ ÉLEVÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.02.2012 EP 12155529**

(43) Veröffentlichungstag der Anmeldung:
**24.12.2014 Patentblatt 2014/52**

(73) Patentinhaber: **BASF SE**
**67056 Ludwigshafen am Rhein (DE)**

(72) Erfinder:
• WEISMANTEL, Matthias
  63637 Jossgrund (DE)
• RIEGEL, Ulrich
  66849 Landstuhl (DE)
• GIEGER, Thomas
  67069 Ludwigshafen (DE)
• BRAUN, Markus
  69118 Heidelberg (DE)
• MITCHELL, Michael
  Waxhaw, NC 28173 (US)

(74) Vertreter: **BASF IP Association**
**BASF SE**
**G-FLP-C006**
**67056 Ludwigshafen (DE)**

(56) Entgegenhaltungen:
EP-A1- 2 399 944       WO-A1-2008/046841
WO-A1-2010/018143      WO-A1-2011/078298
WO-A1-2011/126079      WO-A1-2012/143235
DE-A1- 3 831 261       JP-A- 2002 282 687
JP-A- 2009 052 009     US-A1- 2002 128 618
US-A1- 2002 165 288    US-A1- 2012 001 122

**Beschreibung**

Die vorliegende Erfindung betrifft ein Verfahren zur Herstellung wasserabsorbierender Polymerpartikel mit hoher Quellgeschwindigkeit und hoher Permeabilität durch Polymerisation einer wässrigen Monomerlösung oder -suspension zu einem wässrigen Polymergel, wobei ein thermisches Blähmittel, das im Wesentlichen frei ist von anorganischen Säureanionen, in das Polymergel eingemischt wird, und anschließende thermische Trocknung des Polymergels.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Es besteht die zunehmende Tendenz Hygieneartikel, wie Babywindeln, Inkontinenzartikel, Damenbinden dünner zu machen und den Anteil an großvolumigem Fluff, z. B. Zellulosefasern zu reduzieren. Der Fluff hat u. a. die Aufgabe des Flüssigkeitstransports in der Windel, die wasserabsorbierenden Polymerpartikel müssen somit neben der Aufnahme und Speicherung der Flüssigkeiten auch zunehmend weitere Aufgaben, wie z. B. schnelle Aufnahme, Transport und Verteilung der Flüssigkeiten sicherstellen.

Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Für eine weitere Verbesserung der Anwendungseigenschaften der wasserabsorbierenden Polymerpartikel, wie beispielsweise der Permeabilität des gequollenen Gelbetts (SFC) in der Windel und Absorption unter einem Druck von 49.2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Ferner können hohe Anteile an nicht umgesetzten Monomeren bei der Herstellung wasserabsorbierender Polymerpartikel, die so genannten Restmonomere, toxikologisch bedenklich sein. Um den Restmonomerengehalt zu reduzieren wird in WO 2010/018143 A1 beispielsweise ein Salz des Harnstoffs mit einer anorganischen Säure eingesetzt.

Darüber hinaus kann, um eine schnelle Flüssigkeitsaufnahme zu gewährleisten, die Quellgeschwindigkeit der wasserabsorbierenden Polymerpartikel erhöht werden. Dies wird beispielsweise in DE 38 31 261 beschrieben.

Allerdings hat eine zu hohe Quellgeschwindigkeit einen negativen Einfluss auf die Permeabilität des gequollenen Gelbetts (SFC), diese wird herabgesetzt, so dass dies beispielsweise bei Verwendung z. B. in Babywindeln die Gefahr des Gelblockkings erhöht und somit ein Auslaufen der Windel begünstigt.

Zudem werden, wenn z.B. Harnstoff in die Monomerlösung eingebracht wird, die erhaltenen wasserabsorbierenden Polymerpartikel poröser und dadurch instabiler. Sie können daher z.B. bei der Verwendung bzw. Verarbeitung leicht zerbrechen und verlieren so auch ihre Anwendungseigenschaften und somit ihre Funktionsfähigkeit.

WO 2012/143235 A1 offenbart den Zusatz von Blähmitteln zur Monomerlösung vor dem Start der Polymerisation.

WO 2011/078298 A1 offenbart die Polymerisation von Gasblasen enthaltenden Monomerlösungen.

JP 2009-52009 und JP 2002-282687 offenbaren den Zusatz von Blähmitteln nach der Polymerisation.

Aufgabe der vorliegenden Erfindung war daher die Bereitstellung verbesserter wasserabsorbierender Polymerpartikel, die eine hohe Quellgeschwindigkeit (FSR) bei hoher Permeabilität des Gelbetts (SFC) und hoher CRC aufweisen. Ferner war es Aufgabe der vorliegenden Erfindung wasserabsorbierende Partikel bereitzustellen, die bei der Verwendung und/oder Verarbeitung ihre Anwendungseigenschaften und somit ihre Funktionsfähigkeit behalten.

Die Herstellung der erfindungsgemäßen wasserabsorbierenden Polymerpartikel erfolgt durch Polymerisation einer wässrigen Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

zu einem wässrigen Polymergel, die thermische Trocknung des Polymergels, die Zerkleinerung des getrockneten Polymergels zu Polymerpartikeln und die Klassierung der erhaltenen Polymerpartikel, wobei ein thermisches Blähmittel, das im Wesentlichen frei ist von anorganischen Säureanionen, in das wässrige Polymergel eingemischt wird, die Polymerisation in einem Kneter durchgeführt wird und das thermische Blähmittel nach mindestens 50% der Verweilzeit im Kneter zugesetzt wird, von 0,2 bis 5 Gew.-% des thermischen Blähmittels, bezogen auf das unneutralisierte Monomer a), eingesetzt werden und das Monomer a) zu 30 bis 80 mol-% neutralisiert ist.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 80 mol-%, ganz besonders bevorzugt mindestens 90 mol-%.

Es ist ganz besonders bevorzugt, dass das Monomer a) zu mindestens 90 mol-% Acrylsäure ist Die eingesetzte Acrylsäure enthält üblicherweise Polymerisationsinhibitoren, vorzugsweise Hydrochinonhalbether, als Lagerstabilisator.

Die Monomerlösung enthält daher vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf die unneutralisierte Acrylsäure. Beispielsweise kann zur Herstellung der Monomerlösung eine Acrylsäure mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen der Acrylsäure kovalente Bindungen ausbilden können. Weiterhin sind auch polyvalente Metallsalze, die mit mindestens zwei Säuregruppen der Acrylsäure koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, jeweils bezogen auf das unneutralisierte Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit Acrylsäure copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssiger Acrylsäure, beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Zur Verbesserung der Trocknungseigenschaften kann das mittels eines Kneters erhaltene zerkleinerte Polymergel zusätzlich extrudiert werden.

Das thermische Blähmittel wird bevorzugt nach mindestens 60 % der Verweilzeit, besonders bevorzugt nach mindestens 75% der Verweilzeit im Kneter zugesetzt.

Erfindungsgemäß wird das Blähmittel in eine Zone des Kneters eingespeist, in der sie Polymerisation noch nicht beendet ist. Der Polymerisationsgrad des Gels in dieser Zone beträgt 10 - 99,9%, bevorzugt 55 - 99,5% besonders bevorzugt 90 - 99%.

Die Menge des zugegebenen Blähmittels beträgt bezogen auf das unneutralisierte Monomer a) besonders bevorzugt 0,5 - 2,5 Gew.-%.

Geeignete thermische Blähmittel sind Azoverbindungen, wie z. B. Azodicarbonamid, Azobisisobutyronitril und Diazoaminobenzole, Acrylsäureverbindungen von Azoverbindungen, die Aminogruppen enthalten, Nitroverbindungen, wie beispielsweise Nitroharnstoff, Nitrosoverbindungen, wie Dinitrosopentamethylentetramin, Carbonate, wie z.B. Natriumcarbonat, Ammoniumcarbonat, oder Hydrazine, Sulphohydrazide, Triazinverbindungen, Sulphonyl-semicarbazide, Guanidin-Derivate wie z.B. Aminoguanidincarbonat und Harnstoff, wobei Harnstoff besonders gut geeignet ist.

Die Blähmittel sind im Wesentlichen frei von anorganischen Säureanionen, d.h. die Blähmittel enthalten weniger als 10 Mol-% anorganische Säureanionen.

Anorganische Säureanionen im Sinne dieser Erfindung sind nur Anionen der starken Mineralsäuren, wie Schwefelsäure, Phosphorsäure, Salpetersäure. Die anorganischen Säureanionen können als Gegenion des Blähmittels vorliegen, beispielsweise Phosphat in Harnstoffphosphat.

Die Permeabilität von wasserabsorbierenden Polymerpartikeln ist bei Verwendung von Blähmitteln mit anorganischen Säureanionen niedriger als bei Blähmitteln, die im Wesentlichen frei sind von anorganischen Säureanionen.

Es wird angenommen, dass sich anorganische Säureanionen in die entsprechenden Säuren umwandeln und die so erzeugten anorganischen Säuren die Anzahl der Vernetzungsstellen, insbesondere auf der Oberfläche der wasserabsorbierenden Polymerpartikel beeinflussen, indem sie Esterbindungen der Oberflächennachvernetzungsverbindungen zerstören oder deren Bildung bei der Nachvernetzungsreaktion verhindern.

Es ist zudem auch möglich, dass anorganische Säuren Esterverbindungen des Innenvernetzers zerstören und so den Vernetzungsgrad im Inneren der Partikel beeinflussen.

Die Säuregruppen der erhaltenen Polymergele sind üblicherweise teilweise neutralisiert. Die Neutralisation wird auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt 30 bis 80 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content - Weight Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Erfindungsgemäß erfolgt die thermische Trocknung bei einer Temperatur oberhalb der Zersetzungstemperatur des thermischen Blähmittels. Das thermische Blähmittel spaltet unter Einfluss von Wärme Gase (z. B. $N_2$, im Falle von Harnstoff $CO_2$ und $NH_3$) ab, die so zu einer Porenbildung im Polymergel führen.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der wasserabsorbierenden Polymerpartikel beträgt vorzugsweise mindestens 200 $\mu$m, besonders bevorzugt von 250 bis 600 $\mu$m, ganz besonders von 300 bis 500 $\mu$m. Die mittlere Partikelgröße kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 $\mu$m beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von 150 bis 850 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die klassierten Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft.

Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Quellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Restmonomer

Der Gehalt an Restmonomer der wasserabsorbierenden Polymerpartikel wird gemäß der von der EDANA empfohlenen Testmethode Nr. WSP 210.2-04 "Determination of the Amount of Residual Monomers" bestimmt.

Feuchtegehalt

Der Feuchtegehalt der wasserabsorbierenden Polymerpartikel wird gemäß der von der EDANA empfohlenen Testmethode Nr. WSP 230.2-05 "Moisture Content - Weight Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Gravimetric Determination of Fluid Retention Capacity in Saline Solution, After Centrifugation" bestimmt.

Absorption unter einem Druck von 21,0 g/cm$^2$ (Absorption under Load)

Die Absorption unter einem Druck von 21,0 g/cm$^2$ (AUL0.3psi) der wasserabsorbierenden Polymerpartikel wird gemäß der von der EDANA empfohlenen Testmethode Nr. WSP 242.2-05 " Gravimetric Determination of Absorption Under Pressure, " bestimmt.

Absorption unter einem Druck von 49,2 g/cm$^2$ (Absorption under Load)

Die Absorption unter einem Druck von 49,2 g/cm$^2$ (AUL0.7psi) wird analog der von der EDANA empfohlenen Testmethode Nr. WSP 242.2-05 " Gravimetric Determination of Absorption Under Pressure, " bestimmt, wobei statt eines Drucks von 21,0 g/cm$^2$ (AUL0.3psi) ein Druck von 49,2 g/cm$^2$ (AUL0.7psi) eingestellt wird.

Extrahierbare

Der Gehalt an extrahierbaren Bestandteilen der wasserabsorbierenden Polymerpartikel wird gemäß der von der EDANA empfohlenen Testmethode Nr. WSP 270.2-05 "Determination of Extractable PolymerContent by Potentionmetric Titration " bestimmt.

Quellgeschwindigkeit (Free Swell Rate)

Zur Bestimmung der Quellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$\text{FSR [g/g s]} = W2/(W1 \text{x} t)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Permeabilität (Saline Flow Conductivity)

Die Permeabilität (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Permeabilität (SFC) wird wie folgt berechnet:

$$\text{SFC [cm}^3\text{s/g]} = (Fg(t=0)\text{x}L0)/(d\text{x}A\text{x}WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

Beispiele

Herstellung der Grundpolymere:

Beispiel 1 (Vergleichsbeispiel)

Ein kontinuierlicher Knetreaktor mit zwei Wellen vom Typ List ORP 10 Contikneter (LIST AG, Arisdorf, Schweiz) wurde am Mantel und an beiden Wellen während der gesamten Versuchsdauer konstant auf 95°C beheizt. Die Kneterwellen wurden mit 40 Upm bzw. 10 Upm betrieben. Der Durchsatz an Monomerlösung betrug 39,343 kg/h (Mischung aus 31,913 kg/h einer 37,3gew.-%igen wässrigen Natriumacrylatlösung, 3,682 kg/h Acrylsäure und 3,748 kg/h entsalztem Wasser). Die Monomerlösung wurde mit Stickstoff inertisiert und hatte eine Temperatur von 23°C. Zusätzlich wurden der Monomerlösung über ein T-Stück vor dem Reaktor eine Mischung aus 46,0 g/h 3-fach ethoxyliertem Glycerintriacrylat und 413,9 g/h Acrylsäure sowie 88,1 g/h einer 15gew.-%igen wässrigen Natriumpersulfatlösung und 26,4 g/h einer 1,0gew.-%igen wässrigen Wasserstoffperoxidlösung zugegeben. Nach ¼ der Länge des Knetreaktors (entspricht 25% der durchschnittlichen Verweilzeit) wurden über eine Düse 81,9 g/h einer 0,5gew.-%igen wässrigen Ascorbinsäurelösung zudosiert.

Das ausgetragene Polymergel wurde auf etwa 60°C abgekühlt, in Portionen von je 1000 g gleichmäßig auf 473 cm$^2$ eines Gitterblechs aufgeteilt und in einem Umlufttrockenschrank 90 Minuten bei 175°C getrocknet. Anschließend wurde das getrocknete Polymergel mittels eines Walzenstuhls vom Typ LRC 125/70 (Bauermeister Zerkleinerungstechnik GmbH, Norderstedt, Deutschland) gemahlen, wobei nacheinander Spaltweiten von 1000 μm, 600 μm und 400 μm eingestellt wurden. Die wasserabsorbierenden Polymerpartikel wurden abgesiebt und die erhaltenen Siebfraktionen wurden so abgemischt, dass folgende Partikelgrößenverteilung erhalten wurde:

| | |
|---|---|
| >710 μm | 0 Gew.-% |
| 710 μm - 600 μm | 13,3 Gew.-% |
| 500 μm - 600 μm | 23,3 Gew.-% |
| 300 μm - 500 μm | 43,6 Gew.-% |
| 150 μm - 300 μm | 19,8 Gew.-% |
| < 150 μm | 0 Gew.-%. |

Die erhaltene Mischung wird in einem 5 l Metallgefäß in einem Röhnrad-Mischer vom Typ RRM ELTE 650 ST (Engelsmann AG, Ludwigshafen, Deutschland) homogenisiert.

Die erhaltenen wasserabsorbierenden Polymerpartikel wurden analysiert. Die Ergebnisse sind in Tabelle 1 zusammengefasst.

Beispiel 2 (Vergleichsbeispiel)

Es wurde verfahren wie unter Beispiel 1. Zusätzlich wurden in der Monomerlösung 237,9 g/h Harnstoff gelöst.

Die erhaltenen wasserabsorbierenden Polymerpartikel wurden analysiert. Die Ergebnisse sind in Tabelle 1 zusammengefasst.

Beispiel 3

Es wurde verfahren wie unter Beispiel 1. Zusätzlich wurden während der Polymerisation nach ¾ der Länge (entspricht 75% der durchschnittlichen Verweilzeit) des Knetreaktors über eine Düse 732 g/h einer 32,5gew.-%igen wässrigen Harnstofflösung auf das Polymergel aufgesprüht.

Die erhaltenen wasserabsorbierenden Polymerpartikel wurden analysiert. Die Ergebnisse sind in Tabelle 1 zusammengefasst.

Beispiel 4 (Vergleichsbeispiel)

Es wurde verfahren wie unter Beispiel 1. Zusätzlich wurden während der Polymerisation nach ¾ der Länge (entspricht 75% der durchschnittlichen Verweilzeit) des Knetreaktors über eine Düse 3366,2 g/h einer 20,0 gew.-%igen wässrigen Harnstoffphosphatlösung auf das Polymergel aufgesprüht.

Die erhaltenen wasserabsorbierenden Polymerpartikel wurden analysiert. Die Ergebnisse sind in Tabelle 1 zusammengefasst.

Tabelle 1: Zusammenfassung der Grundpolymere (alle Werte Restfeuchte-korrigiert)

| Beispiel | CRC [g/g] | AUL0.3psi [g/g] | Feuchtegehalt [Gew.-%] | Extrahierbare [Gew.-%] |
|----------|-----------|-----------------|------------------------|------------------------|
| 1*) | 38,9 | 8,8 | 3,3 | 16,0 |
| 2*) | 39,1 | 8,8 | 2,5 | 17,3 |
| 3 | 37,7 | 9,1 | 3,2 | 15,3 |
| 4*) | 34,9 | 11,3 | 2,7 | 15,7 |
| *) Vergleichsbeispiel | | | | |

Oberflächennachvernetzung der Grundpolymere:

Beispiel 5 (Vergleichsbeispiel)

1200 g des Grundpolymers aus Beispiels 1 wurden in einem Pflugschar®-Mischer mit Heizmantel vom Typ M5 (Gebr. Lödige Maschinenbau GmbH, Paderborn, Deutschland) bei 23°C und einer Wellendrehzahl von 200 Upm mittels einer Zweistoff-Sprühdüse mit folgender Lösung beschichtet (jeweils bezogen auf das Grundpolymer):

| | |
|---|---|
| 0,992 Gew.-% | Isopropanol |
| 0,07 Gew.-% | N-(2-Hydroxyethyl)-2-oxazolidinon |
| 0,07 Gew.-% | 1,3-Propandiol |
| 0,248 Gew-% | entsalztes Wasser |
| 0,70 Gew.-% | 1,2-Propandiol |
| 2,72 Gew.-% | 22gew.-%ige wässrige Aluminiumlaktatlösung |
| 0,20 Gew.-% | 2gew.-%ige wässrige Sorbitanmonococcoatlösung |

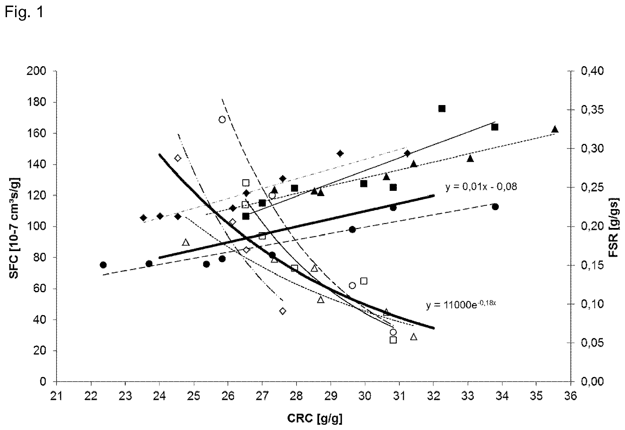

Nach dem Aufsprühen wurde die Wellendrehzahl auf 50 Upm reduziert und das Produkt durch Erhöhung der Temperatur des Heizmantels (Temperatur der Heizflüssigkeit 238°C) auf eine Produkttemperatur von 185°C gebracht. Dem Reaktionsgemisch wurden alle 5 Minuten von jeweils etwa 20 g, beginnend mit dem Erreichen der Produkttemperatur von 185°C entnommen. Die Proben wurden jeweils auf 23°C abkühlen gelassen und auf < 710 μm abgesiebt.
Die erhaltenen oberflächennachvernetzten wasserabsorbierenden Polymerpartikel wurden analysiert. Die Ergebnisse wurden in Tabelle 2 und Figur 1 zusammengefasst.

Beispiel 6 (Vergleichsbeispiel)

Es wurde verfahren wie unter Beispiel 5, wobei 1200 g des Grundpolymers aus Beispiel 2 eingesetzt wurden
Die erhaltenen oberflächennachvernetzten wasserabsorbierenden Polymerpartikel wurden analysiert. Die Ergebnisse wurden in Tabelle 2 und Figur 1 zusammengefasst.

Beispiel 7

Es wurde verfahren wie unter Beispiel 5, wobei 1200 g des Grundpolymers aus Beispiel 3 eingesetzt wurden
Die erhaltenen oberflächennachvernetzten wasserabsorbierenden Polymerpartikel wurden analysiert. Die Ergebnisse wurden in Tabelle 2 und Figur 1 zusammengefasst.

Beispiel 8 (Vergleichsbeispiel)

Es wurde verfahren wie unter Beispiel 5, wobei 1200 g des Grundpolymers aus Beispiel 4 eingesetzt wurden
Die erhaltenen oberflächennachvernetzten wasserabsorbierenden Polymerpartikel wurden analysiert. Die Ergebnisse wurden in Tabelle 2 und Figur 1 zusammengefasst.

Tabelle 2. Zusammenfassung der oberflächennachvernetzten Grundpolymere

| Beispiel | Zeit [min] | SFC [$10^{-7}$ cm$^3$s/g] | CRC [g/g] | AUL0.7psi [g/g] | FSR [g/gs] |
|---|---|---|---|---|---|
| 5*) | 25 | 0 | 33,8 | 16,4 | 0,23 |
| | 30 | 32 | 30,8 | 23,0 | 0,22 |
| | 35 | 62 | 29,6 | 23,6 | 0,20 |
| | 40 | 120 | 27,3 | 24,2 | 0,16 |
| | 45 | 169 | 25,8 | 23,4 | 0,16 |
| | 50 | | 25,4 | 22,8 | 0,15 |
| | 55 | | 23,7 | 22,1 | 0,15 |
| | 60 | | 22,4 | 21,6 | 0,15 |
| 6*) | 25 | 0 | 35,6 | 10,3 | 0,33 |
| | 30 | 0 | 33,1 | 17,6 | 0,29 |
| | 35 | 29 | 31,4 | 22,7 | 0,28 |
| | 40 | 45 | 30,6 | 24,1 | 0,26 |
| | 45 | 53 | 28,7 | 24,4 | 0,24 |
| | 50 | 73 | 28,5 | 24,3 | 0,25 |
| | 55 | 79 | 27,4 | 23,6 | 0,25 |
| | 60 | 90 | 24,8 | 23,1 | |
| 7 | 25 | | 33,8 | 12,9 | 0,33 |
| | 30 | | 32,2 | 19,9 | 0,35 |
| | 35 | 27 | 30,8 | 23,9 | 0,25 |
| | 40 | 65 | 30,0 | 24,3 | 0,26 |
| | 45 | 73 | 27,9 | 23,6 | 0,25 |
| | 50 | 94 | 27,0 | 23,4 | 0,23 |
| | 55 | 114 | 26,5 | 23,5 | 0,22 |
| | 60 | 128 | 26,5 | 23,0 | 0,21 |
| 8*) | 25 | | 31,2 | 14,8 | 0,29 |
| | 30 | | 29,3 | 19,4 | 0,29 |
| | 35 | 45 | 27,6 | 20,3 | 0,26 |
| | 40 | 85 | 26,5 | 21,1 | 0,24 |
| | 45 | 103 | 26,1 | 20,9 | 0,22 |
| | 50 | 144 | 24,5 | 20,2 | 0,21 |
| | 55 | | 24,0 | 20,2 | 0,21 |
| | 60 | | 23,5 | 19,9 | 0,21 |
| *) Vergleichsbeispiel | | | | | |

In Figur 1 bedeuten die leeren Kreise (Beispiel 5), Dreiecke (Beispiel 6),Quadrate (Beispiel 7) und Rauten (Beispiel 8) jeweils die Permeabilität (SFC) und die gefüllten Kreise (Beispiel 5), Dreiecke (Beispiel 6), Quadrate (Beispiel 7) und Rauten (Beispiel 8) jeweils die Quellgeschwindigkeit (FSR).

**Patentansprüche**

1.  Verfahren zur Herstellung wasserabsorbierender Polymerpartikel, umfassend die Polymerisation einer wässrigen Monomerlösung oder -suspension, enthaltend

    a) mindestens ein ethylenisch ungesättigtes, säuregruppentragendes Monomer, das zumindest teilweise neutralisiert sein kann,
    b) mindestens einen Vernetzer,
    c) mindestens einen Initiator,
    d) optional ein oder mehrere mit den unter a) genannten Monomeren copolymerisierbare ethylenisch ungesättigte Monomere und

e) optional ein oder mehrere wasserlösliche Polymere,

zu einem wässrigen Polymergel, die thermische Trocknung des Polymergels, die Zerkleinerung des getrockneten Polymergels zu Polymerpartikeln und die Klassierung der erhaltenen Polymerpartikel, **dadurch gekennzeichnet, dass** ein thermisches Blähmittel, das im Wesentlichen frei von anorganischen Säureanionen ist, in das wässrige Polymergel eingemischt wird, die Polymerisation in einem Kneter durchgeführt wird und das thermische Blähmittel nach mindestens 50% der Verweilzeit im Kneter zugesetzt wird, von 0,2 bis 5 Gew.-% des thermischen Blähmittels, bezogen auf das unneutralisierte Monomer a), eingesetzt werden und das Monomer a) zu 30 bis 80 mol-% neutralisiert ist.

2. Verfahren gemäß Anspruch 1, **dadurch gekennzeichnet, dass** von 0,5 bis 2,5 Gew.-% des thermischen Blähmittels, bezogen auf das unneutralisierte Monomer a), eingesetzt werden.

3. Verfahren gemäß einem der Ansprüche 1 oder 2, **dadurch gekennzeichnet, dass** das thermische Blähmittel Harnstoff ist.

4. Verfahren gemäß einem der Ansprüche 1 bis 3, **dadurch gekennzeichnet, dass** die thermische Trocknung bei einer Temperatur oberhalb der Zersetzungstemperatur des thermischen Blähmittels durchgeführt wird.

5. Verfahren gemäß einem der Ansprüche 1 bis 4, **dadurch gekennzeichnet, dass** das Monomer a) zu mindestens 90 mol-% Acrylsäure ist.

6. Verfahren gemäß einem der Ansprüche 1 bis 5, **dadurch gekennzeichnet, dass** die Monomerlösung oder -suspension, bezogen auf das unneutralisierte Monomer a) von 0,1 bis 1 Gew.-% des Vernetzers b) enthält.

7. Verfahren gemäß einem der Ansprüche 1 bis 6, **dadurch gekennzeichnet, dass** die klassierten Polymerpartikel oberflächennachvernetzt werden.

## Claims

1. A process for producing water-absorbing polymer particles, comprising the polymerization of an aqueous monomer solution or suspension comprising

   a) at least one ethylenically unsaturated monomer which bears acid groups and may be at least partly neutralized,
   b) at least one crosslinker,
   c) at least one initiator,
   d) optionally one or more ethylenically unsaturated monomers copolymerizable with the monomers mentioned under a) and
   e) optionally one or more water-soluble polymers,

   to give an aqueous polymer gel, the thermal drying of the polymer gel, the comminution of the dried polymer gel to polymer particles and the classification of the resulting polymer particles, wherein a thermal blowing agent essentially free of inorganic acid anions is mixed into the aqueous polymer gel, the polymerization is performed in a kneader and the thermal blowing agent is added after at least 50% of the residence time in the kneader, from 0.2 to 5% by weight of the thermal blowing agent, based on the unneutralized monomer a), is used and monomer a) has been neutralized to an extent of 30 to 80 mol%.

2. The process according to claim 1, wherein from 0.5 to 2.5% by weight of the thermal blowing agent, based on the unneutralized monomer a), is used.

3. The process according to either of claims 1 and 2, wherein the thermal blowing agent is urea.

4. The process according to any of claims 1 to 3, wherein the thermal drying is performed at a temperature above the decomposition temperature of the thermal blowing agent.

5. The process according to any of claims 1 to 4, wherein monomer a) is acrylic acid to an extent of at least 90 mol%.

6. The process according to any of claims 1 to 5, wherein the monomer solution or suspension, based on the unneutralized monomer a), comprises from 0.1 to 1% by weight of the crosslinker b).

7. The process according to any of claims 1 to 6, wherein the classified polymer particles are surface postcrosslinked.

**Revendications**

1. Procédé de fabrication de particules polymères absorbant l'eau, comprenant la polymérisation d'une solution ou suspension aqueuse de monomères, contenant :

a) au moins un monomère éthyléniquement insaturé portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères indiqués en a), et
e) éventuellement un ou plusieurs polymères solubles dans l'eau,

en un gel polymère aqueux, le séchage thermique du gel polymère, le broyage du gel polymère séché en particules polymères, et la classification des particules polymères obtenues, **caractérisé en ce qu'**un agent gonflant thermique, qui est essentiellement exempt d'anions acides inorganiques, est incorporé dans le gel polymère aqueux, la polymérisation est réalisée dans un malaxeur et l'agent gonflant thermique est ajouté après au moins 50 % du temps de séjour dans le malaxeur, de 0,2 à 5 % en poids de l'agent gonflant thermique, par rapport au monomère a) non neutralisé, est utilisé, et le monomère a) est neutralisé à hauteur de 30 à 80 % en moles.

2. Procédé selon la revendication 1, **caractérisé en ce que** de 0,5 à 2,5 % en poids de l'agent gonflant thermique, par rapport au monomère a) non neutralisé, est utilisé.

3. Procédé selon l'une quelconque des revendications 1 ou 2, **caractérisé en ce que** l'agent gonflant thermique est l'urée.

4. Procédé selon l'une quelconque des revendications 1 à 3, **caractérisé en ce que** le séchage thermique est réalisé à une température supérieure à la température de décomposition de l'agent gonflant thermique.

5. Procédé selon l'une quelconque des revendications 1 à 4, **caractérisé en ce que** le monomère a) est l'acide acrylique à hauteur d'au moins 90 % en moles.

6. Procédé selon l'une quelconque des revendications 1 à 5, **caractérisé en ce que** la solution ou suspension de monomères contient, par rapport au monomère a) non neutralisé, de 0,1 à 1 % en poids de l'agent de réticulation b).

7. Procédé selon l'une quelconque des revendications 1 à 6, **caractérisé en ce que** les particules polymères classifiées sont post-réticulées en surface.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010018143 A1
- DE 3831261
- WO 2012143235 A1
- WO 2011078298 A1
- JP 2009052009 A
- JP 2002282687 A
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- Standard Test Methods for the Nonwovens Industry. Worldwide Strategic Partners. 2005, vol. 157, 1030